# FASCICULE DE BREVET EUROPEEN

(11) **EP 3 666 326 B1**
(45) Date de publication et mention de la délivrance du brevet: **30.07.2025**
(21) Numéro de dépôt: 19214154.7
(22) Date de dépôt: 06.12.2019
(51) Int. Cl.: A61N 1/02, A61N 1/372, G16H 40/67, G06F 21/00

(54) **SYSTÈME ET MÉTHODE D'ÉCRITURE DANS LA MÉMOIRE D'UN DISPOSITIF MÉDICAL ACTIF IMPLANTABLE PAR TÉLÉMÉTRIE**
SYSTEM UND METHODE ZUM SCHREIBEN DURCH FERNÜBERTRAGUNG IN EINEN SPEICHER EINES IMPLANTIERBAREN AKTIVEN MEDIZINPRODUKTS
SYSTEM AND METHOD FOR WRITING IN THE MEMORY OF AN ACTIVE IMPLANTABLE MEDICAL DEVICE BY TELEMETRY

(30) Priorité: 11.12.2018 FR 1872671
(43) Date de publication de la demande: 17.06.2020
(73) Titulaire: Sorin CRM SAS, 92140 Clamart (FR)
(72) Inventeur: LEGAY, Thierry, 91640 Fontenay les Briis (FR); HERY, Laure, 54370 Maixe (FR)
(74) Mandataire: Ungerer, Olaf

(56) Documents cités:
- WO-A1-2009/023328
- WO-A2-2012/092189
- US-A1- 2005 113 885
- US-A1- 2010 076 522
- US-A1- 2014 304 773
- US-A1- 2015 089 590
- US-B1- 6 510 345

## Description

La présente invention concerne un ensemble de dispositif ainsi qu'une méthode pour écrire à distance dans la mémoire d'un dispositif médical actif implantable.

Un dispositif médical implantable actif, par exemple un stimulateur cardiaque implantable ou un système de neurostimulation cérébrale, comprend généralement une fonction de télémétrie permettant une communication par des moyens non-invasifs avec d'autres dispositifs ou moniteurs afin de transmettre des informations et des données d'un dispositif à l'autre. Des exemples de fonctions de télémétrie comprennent notamment la programmation du dispositif implantable pour effectuer certaines tâches de surveillance ou thérapeutiques, la transmission de données physiologiques acquises en temps réel par le dispositif implantable et/ou la mise à jour du programme ou du logiciel du dispositif médical implantable.

La communication par télémétrie entre le dispositif médical implantable actif et un autre dispositif externe peut s'effectuer selon un premier mode de communication, celui de la télémétrie à champ proche (« Near Field Telemetry » en anglais) ou de la télémétrie inductive. Ce premier mode de communication est basé sur le couplage inductif entre deux bobines positionnées étroitement en regard l'une de l'autre en utilisant l'inductance mutuelle entre ces bobines. Ce premier mode de communication nécessite un boîtier de télémétrie inductive contenant une bobine et connecté via un fil électrique à un moniteur externe, boîtier qu'il faut placer et maintenir au-dessus de la bobine du dispositif médical implantable actif afin de permettre la communication entre les deux appareils par inductance. De ce fait, la mobilité du patient est réduite lors du transfert des données car la télémétrie inductive est à courte distance, de l'ordre de cinq à dix centimètres, et nécessite alors une proximité étroite entre le dispositif implanté dans le patient et le boîtier de télémétrie inductive. De plus, la télémétrie inductive a une vitesse de transfert de données plutôt lente, de l'ordre de plusieurs kilobits par seconde. Cette vitesse de transfert n'est pas adaptée pour les dispositifs médicaux implantables actifs modernes, qui peuvent contenir des millions de bits de données physiologiques du patient.

Pour les applications de télésurveillance, les dispositifs médicaux implantables actifs plus récents ont recours à un autre type de mode de communication dit de télémétrie de longue portée, ce qui comprend entre autre, par exemple, la télémétrie sans fil (« Wireless Telemetry » en anglais) utilisant une connexion réseau (par exemple le réseau internet ou une ligne téléphonique), et les systèmes de télémétrie utilisant des rayonnements électromagnétiques de champ éloigné (« far-field electromagnetic radiation » en anglais). Le document US 2004/0260363 A1 se rapporte à une telle télémétrie à longue portée permettant à un dispositif implantable de transmettre des données à une unité de surveillance à distance et d'être programmé par un clinicien situé à un emplacement distant du patient. Ainsi, la télémétrie longue portée permet aux cliniciens depuis un centre hospitalier de surveiller et d'effectuer un suivi des patients qui sont restés à leur domicile, ou même, qui se trouvent ailleurs dans le monde. La télémétrie de longue portée offre ainsi davantage de mobilité au patient lors du transfert de donnée, et fournit également un taux de transfert de données plus élevé, ce qui permet de réduire le temps de téléchargement.

Néanmoins, pour des raisons de confidentialité et de sécurité, et notamment en cas de piratage du système de télémétrie, l'usage de la télémétrie de longue portée est généralement limité au téléchargement des données à partir du dispositif médical implantable actif, par exemple pour effectuer un suivi à distance, et n'est pas appliqué à la modification ou la mise à jour logiciel du dispositif implantable, c'est-à-dire de l'écriture de la mémoire du dispositif médical implantable.

Afin de contrecarrer le risque de piratage de la télémétrie de longue portée entre le dispositif médical implantable du patient et dispositif externe du clinicien (par exemple une unité de contrôle), le document US 2004/0260363 A1 cité ci-dessus suggère de mettre en œuvre un système de verrouillage et d'authentification basé sur de la télémétrie à courte portée en utilisant le champ magnétique statique d'un aimant pour autoriser l'accès à distance au dispositif implantable et de crypter les communications de télémétrie longue portée.

Cependant, des préoccupations au sujet de la sécurité d'un tel système subsistent à cause de l'accès direct par télémétrie de longue portée entre le dispositif médical implantable et un dispositif externe de programmation.

Le document US 2007/0135855 A1 concerne un dispositif de programmation portable capable de programmer une pluralité de dispositifs médicaux implantables conjointement ou indépendamment, et qui est configuré pour interagir simultanément avec une gamme de périphériques externes, tel qu'un serveur ou un ordinateur. Le dispositif de programmation portable selon US 2007/0135855 A1 fonctionne comme un canal de communication entre les dispositifs médicaux implantables et un dispositif externe via l'interface d'un portail de réseau, en particulier un réseau internet. Lorsque la permission d'accéder au dispositif médical implantable est délivrée, suite à l'authentification des identifiants enregistrés dans le dispositif de programmation portable, le téléchargement et le traitement de données par l'intermédiaire du dispositif de programmation portable entre chaque dispositif médical implantable et le dispositif externe sont permis.

Néanmoins, le dispositif de programmation portable selon US 2007/0135855 A1 est limité à un usage en clinique, en particulier pour une utilisation à grande échelle sur plusieurs patients de manière simultanée, et n'est ainsi pas adapté pour un usage au domicile d'un patient qui se trouverait à un endroit éloigné d'un centre médical.

Le document WO 2012/092189 A2 concerne un système et procédé d'interfaçage avec un dispositif médical. Le système comprend un dispositif hôte et un module de communication. Le dispositif hôte a une interface utilisateur configurée pour entrer et afficher des informations relatives à l'interfaçage avec le dispositif médical. Le module de communication est couplé localement au dispositif hôte et configuré pour communiquer sans fil avec le dispositif médical. Le système, mis en œuvre par le dispositif hôte et le module de communication, est configuré pour communiquer avec le dispositif médical doté de fonctions.

Le document US 2005/113885 A1 concerne des techniques pour notifier à un patient le lancement d'une session de télémétrie avec un dispositif médical afin de surveiller ou de reprogrammer des paramètres du dispositif médical.

Il y a donc dans le milieu des dispositifs médicaux actifs implantables un besoin d'améliorer les solutions qui permettent la reprogrammation et l'écriture de la mémoire de tels dispositifs par télémétrie de longue portée, en particulier par connexion réseau, d'un patient se trouvant à son domicile par un clinicien opérant dans un centre médical éloigné de plusieurs kilomètres du domicile du patient.

Par conséquent, l'objet de la présente invention est de proposer un système et une méthode permettant une opération sécurisée d'écriture de la mémoire d'un dispositif médical actif implantable d'un patient se trouvant éloigné de plusieurs kilomètres du clinicien opérant l'opération d'écriture de la mémoire du dispositif implantable.

L'objet de la présente invention est atteint par un système de communication pour permettre une opération d'écriture dans la mémoire d'un dispositif médical actif implantable comprenant : ledit dispositif médical actif implantable qui comprend au moins un capteur de proximité; un dispositif principal d'écriture à distance non-implantable; un dispositif intermédiaire de proximité externe non-implantable qui est configuré pour recevoir des instructions et données d'écriture transmises par le dispositif principal d'écriture à distance par une connexion réseau; le dispositif intermédiaire de proximité étant configuré pour communiquer sans fil avec le dispositif médical implantable, un premier outil de déverrouillage externe non-implantable, distinct du dispositif intermédiaire de proximité, qui est configuré pour émettre un signal détectable par le capteur de proximité lorsque le premier outil de déverrouillage est localisé dans un périmètre prédéterminé du dispositif médical; dans lequel le dispositif intermédiaire de proximité est configuré pour écrire dans la mémoire du dispositif médical actif implantable selon les instructions transmises par le dispositif d'écriture principal à distance en réponse à une détection de la présence dans le périmètre prédéterminé du premier outil de déverrouillage par le capteur de proximité du dispositif médical actif implantable.

De ce fait, ce système permet d'améliorer la sécurité de la communication à distance avec un dispositif médical actif implantable grâce à une première barrière de sécurité établie par le dispositif intermédiaire qui empêche un accès direct du dispositif d'écriture au dispositif médical implantable par connexion réseau ; ainsi qu'une seconde barrière de sécurité grâce au premier outil de déverrouillage qui autorise l'accès au dispositif médical implanté lorsqu'il est localisé dans un périmètre au voisinage immédiat du dispositif médical implantable. Ainsi, l'opération d'écriture ne peut se faire que sur l'autorisation du patient qui doit déverrouiller l'accès de manière active à son dispositif médical implantable par l'usage du premier outil de déverrouillage. Cela permet d'améliorer la sécurité de la télémétrie de longue portée par connexion réseau et ainsi d'optimiser l'écriture de la mémoire d'un dispositif médical implanté par un dispositif d'écriture d'un clinicien qui se trouve éloigné de plusieurs kilomètres du domicile du patient implanté.

La présente invention se rapportant à un système de communication peut être davantage améliorée grâce aux modes de réalisation suivants.

Selon un mode de réalisation, le système peut en outre comprendre un deuxième outil de déverrouillage configuré pour autoriser la communication par radiofréquence entre le dispositif médical actif implantable et le dispositif intermédiaire de proximité lorsque le dispositif intermédiaire de proximité a identifié le deuxième outil de déverrouillage de sorte que le dispositif intermédiaire de proximité est configuré pour écrire dans la mémoire du dispositif médical actif implantable selon les instructions transmises par le dispositif d'écriture principal à distance en réponse à l'identification du deuxième outil de déverrouillage par le dispositif intermédiaire de proximité. La sécurité de l'opération d'écriture peut ainsi être davantage améliorée grâce au deuxième outil de déverrouillage qui permet de maximiser le nombre de barrières de sécurité permettant l'accès au dispositif médical implantable.

Selon un mode de réalisation, le deuxième outil de déverrouillage peut comprendre au moins un mot de passe entré sur le dispositif intermédiaire de proximité, une entrée biométrique reçue par le dispositif intermédiaire de proximité, une carte magnétique lue par le dispositif intermédiaire de proximité, une communication sans fil de courte distance qui émet un signal détectable par un capteur de proximité du dispositif intermédiaire de proximité, un champ magnétique détecté par le dispositif intermédiaire de proximité ou une transmission reçue par l'intermédiaire d'un port périphérique du dispositif intermédiaire de proximité, en particulier un port USB. De ce fait, le deuxième outil de déverrouillage est un outil de déverrouillage de proximité pour un usage dans le voisinage immédiat du dispositif médical implantable. Le deuxième outil de déverrouillage est ainsi tel que son piratage n'est pas possible via une télémétrie de longue portée par exemple.

Selon un mode de réalisation, le dispositif intermédiaire de proximité peut comprendre un moyen de signalisation pour fournir un signal visuel et/ou sonore et/ou vibrant indiquant qu'une opération d'écriture doit être effectuée. Ce moyen de signalisation permet d'alerter le patient qu'une opération d'écriture être effectuée et ainsi de maximiser les chances qu'une requête de réécriture soit détectée par le patient.

Selon un mode de réalisation, le dispositif intermédiaire de proximité peut comprendre au moins un moyen de stockage pour stocker les données d'écriture transmises par le dispositif d'écriture principal à distance au moins jusqu'à ce que l'opération d'écriture est activée. De ce fait, les données d'écriture sont enregistrées et conservée au moins le temps que l'opération d'écriture ne soit autorisée. Cela évite ainsi que les instructions et les données d'écriture fournies par le clinicien au dispositif d'écriture principale ne soient perdues pendant l'attente de l'identification du ou des outils de déverrouillage.

Selon un mode de réalisation, le premier outil de déverrouillage peut être un aimant permanent ou un électroaimant dont le champ magnétique statique est détecté par le capteur de proximité du dispositif médical actif implantable. Le premier outil de verrouillage est ainsi facile à fabriquer et s'avère peu onéreux.

Selon un mode de réalisation, le dispositif intermédiaire de proximité non-implantable peut être un smartphone, une tablette numérique, un dispositif portable connecté, un ordinateur, un dispositif d'équipement médical à domicile ou un système domotique. Ainsi, le dispositif intermédiaire de proximité est, en particulier, un dispositif portable ce qui améliore la mobilité du patient lors de l'opération d'écriture depuis son domicile. En combinaison ou alternativement, le dispositif intermédiaire de proximité peut être utilisé à d'autres fins que celle de l'opération d'écriture, ce qui permet de réduire le nombre de dispositifs au domicile d'un patient.

Selon un mode de réalisation, le système peut en outre comprendre un dispositif de communication portatif, en particulier à un téléphone portable, une tablette, un ordinateur portable et/ou une montre connectée ; auquel la requête pour une opération d'écriture est envoyée par connexion réseau à via un message, un SMS (pour « Short Message Service » en anglais), un email ou un type d'alerte analogue. Ainsi, si la requête envoyée au dispositif intermédiaire de proximité n'a pas été détecté par le patient, l'envoi de la requête à un autre dispositif que le patient est susceptible de garder près de ou sur lui augmente la probabilité que le patient réponde et autorise l'accès à son dispositif médical implanté par télémétrie au clinicien. Ainsi, le nombre de tentatives échouées pour non-réponse à la requête d'une opération d'écriture par le clinicien peut être diminué.

Les modes de réalisation peuvent être combinés pour former davantage de variantes de mode de réalisation avantageux de la présente invention.

L'objet de la présente invention est également atteint par une méthode pour permettre une opération d'écriture dans la mémoire d'un dispositif médical actif implantable comprenant les étapes de: a) envoyer à un dispositif non-implantable intermédiaire de proximité sans fil une requête par connexion réseau pour une opération d'écriture du dispositif médical actif implantable par un dispositif d'écriture principal à distance; b) recevoir par le dispositif intermédiaire de proximité des instructions et des données d'écriture envoyées par connexion réseau par le dispositif d'écriture principal à distance; c) placer un premier outil de déverrouillage externe non-implantable dans un périmètre de proximité prédéterminé du dispositif médical actif implantable afin d'autoriser une communication par radiofréquence entre le dispositif intermédiaire de proximité et le dispositif médical actif implantable ; d) écrire lesdites données d'écriture dans la mémoire du dispositif médical actif implantable par le dispositif intermédiaire de proximité.

Cette méthode permet d'améliorer la sécurité de la communication à distance avec un dispositif médical actif implantable grâce à une première barrière de sécurité établie par le dispositif intermédiaire qui empêche un accès direct du dispositif d'écriture au dispositif médical implantable par connexion réseau ; ainsi qu'une seconde barrière de sécurité grâce au premier outil de déverrouillage qui autorise l'accès au dispositif médical implanté par l'intermédiaire du dispositif de communication intermédiaire. De plus, l'opération d'écriture ne peut se faire que sur la requête d'un clinicien et l'acceptation de cette requête par un patient qui doit alors autoriser l'accès à son dispositif médical implantable par l'usage du premier et du deuxième outil de déverrouillage dans le voisinage immédiat du dispositif médical implantable. La succession de ces étapes permet d'améliorer davantage la sûreté des échanges par connexion réseau. Ainsi, l'amélioration de la sécurité de la télémétrie de longue portée par connexion réseau permet d'optimiser l'écriture de la mémoire d'un dispositif médical implanté par un dispositif d'écriture d'un clinicien qui se trouve éloigné de plusieurs kilomètres du domicile du patient implanté.

La présente invention se rapportant à une méthode pour permettre une opération d'écriture dans la mémoire d'un dispositif médical actif implantable peut être davantage améliorée grâce aux modes de réalisation suivants.

Selon un mode de réalisation, l'opération d'écriture du dispositif médical actif implantable peut être empêchée lorsque le premier outil de déverrouillage n'est pas détecté par le dispositif médical actif implantable, en particulier lorsque la distance entre le premier outil de déverrouillage et le dispositif médical actif implantable est plus grande que 15 centimètres, plus en particulier plus grande que 10 centimètres. De ce faite, le premier outil de déverrouillage nécessite un contact très proche avec le dispositif médical actif implantable, qui peut même être en contact avec la peau du patient, ce qui implique que le patient est conscient de cette étape de déverrouillage et dont qu'il a supposément accepté la requête d'opération d'écriture. Un piratage de cette opération de déverrouillage n'est ainsi pas possible depuis une télémétrie de longue portée.

Selon un mode de réalisation, l'étape b) peut être précédée d'une étape pour recevoir, via le dispositif intermédiaire de proximité, la requête d'autoriser la communication par radiofréquence entre le dispositif intermédiaire de proximité et le dispositif médical actif implantable au moyen d'un deuxième outil de déverrouillage externe non-implantable. La sécurité de l'étape de l'opération d'écriture peut ainsi être davantage améliorée grâce au deuxième outil de déverrouillage qui permet de maximiser le nombre de barrières de sécurité permettant l'accès au dispositif médical implantable.

Selon un mode de réalisation, le second outil de déverrouillage peut comprendre au moins un mot de passe entré sur le dispositif de communication intermédiaire, une entrée biométrique reçue le dispositif de communication intermédiaire, une carte magnétique lue par le dispositif de communication intermédiaire, une communication sans fil de courte distance qui émet un signal détectable par un capteur de proximité du dispositif de communication intermédiaire, un champ magnétique détecté par le dispositif de communication non-implantable ou une transmission reçue par l'intermédiaire d'un port périphérique du dispositif de communication intermédiaire, en particulier un port USB. L'utilisation d'un second outil de déverrouillage permet d'améliorer davantage la sécurité de la communication par connexion réseau en multipliant les requêtes de validation pour autoriser l'opération d'écriture de la mémoire du dispositif médical implantable.

Selon un mode de réalisation, la communication par radiofréquence entre le dispositif médical actif implantable et le dispositif de communication intermédiaire peut être réalisée lorsque lesdits dispositifs sont distants l'un de l'autre de moins de 10 mètres. Les distances permettant la communication entre le dispositif médical actif implantable et le dispositif de communication intermédiaire sont ainsi adaptées pour qu'un patient puisse réaliser l'opération d'écriture à son domicile.

Selon un mode de réalisation, à l'étape b) le premier outil de déverrouillage peut émettre un signal au dispositif médical actif implantable qui provient d'un champ électromagnétique ou d'un champ magnétique statique ou d'un champ inductif. Le premier outil de verrouillage est ainsi facile à fabriquer et s'avère peu onéreux.

Selon un mode de réalisation, la requête pour une opération d'écriture peut être signalée par un moyen de signalisation du dispositif intermédiaire de proximité, qui émet un signal visuel et/ou sonore et/ou vibrant indiquant qu'une opération d'écriture doit être effectuée. Ce moyen de signalisation permet d'alerter le patient qu'une opération d'écriture être effectué et ainsi de maximiser les chances qu'une requête de réécriture soit détectée par le patient.

Selon un mode de réalisation, la requête pour une opération d'écriture peut également être envoyée par connexion réseau à un dispositif de communication portatif, en particulier à un téléphone portable, une tablette, un ordinateur portable et/ou une montre connectée via un message, un SMS (pour « Short Message Service » en anglais), un email ou un type d'alerte analogue. Ainsi, si la requête envoyée au dispositif intermédiaire de proximité n'a pas été détecté par le patient, l'envoi de la requête à un autre dispositif que le patient est susceptible de garder près de ou sur lui augmente la probabilité que le patient réponde et autorise l'accès à son dispositif médical implanté par télémétrie au clinicien. Ainsi, le nombre de tentatives échouées pour non-réponse à la requête d'une opération d'écriture par le clinicien peut être diminué.

Selon un mode de réalisation, les données d'écriture sont stockées par un moyen de stockage du dispositif intermédiaire de proximité au moins jusqu'à l'autorisation d'une communication par radiofréquence entre le dispositif intermédiaire de proximité et le dispositif médical actif implantable. De ce fait, les données d'écriture sont enregistrées et conservée au moins le temps que l'opération d'écriture ne soit autorisée. Cela évite ainsi que les instructions et les données d'écriture fournie par le clinicien au dispositif d'écriture principale aux étapes précédentes a) et b) ne soient perdues pendant l'attente de l'identification du ou des outils de déverrouillage.

Les modes de réalisation peuvent être combinés pour former davantage de variantes de mode de réalisation avantageux de la présente invention.

L'invention et ses avantages seront expliqués plus en détail dans la suite au moyen de modes de réalisation préférés et en s'appuyant notamment sur les figures d'accompagnement suivantes, dans lesquelles :
La Figure 1 représente schématiquement un système de communication pour permettre une opération d'écriture dans la mémoire d'un dispositif médical actif implantable selon un premier mode de réalisation de l'invention ;
La Figure 2 représente schématiquement un système de communication pour permettre une opération d'écriture dans la mémoire d'un dispositif médical actif implantable selon un deuxième mode de réalisation de l'invention ;
La Figure 3 représente un organigramme de la méthode de communication pour permettre une opération d'écriture dans la mémoire d'un dispositif médical actif implantable selon le premier mode de réalisation de l'invention ;
La Figure 4 représente un organigramme de la méthode de communication pour permettre une opération d'écriture dans la mémoire d'un dispositif médical actif implantable selon le deuxième mode de réalisation de l'invention.

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en œuvre de la présente invention.

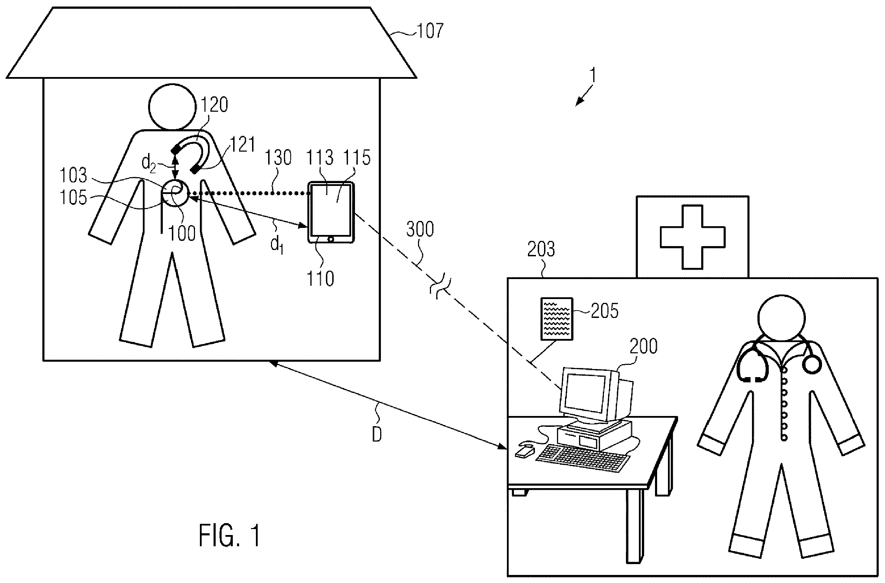

La Figure 1 illustre schématiquement le système de communication 1 pour permettre une opération d'écriture d'un dispositif médical actif implantable 100 selon un premier mode de réalisation de l'invention.

Le dispositif médical implantable 100, tel un stimulateur cardiaque implantable ou un système de neurostimulation cérébrale, est configuré pour être implanté dans un patient. Le dispositif médical implantable 100 comprend, entre autre, un capteur de proximité 103 est une mémoire 105. Des mises à jour du dispositif médical implantable 100, notamment du logiciel de dispositif médical implantable 100, peuvent s'avérer nécessaire au cours de la vie du patient. Afin d'éviter que le patient ne doive se déplacer à un centre hospitalier 203 pour qu'un clinicien procède à une opération d'écriture de la mémoire 105 du dispositif médical implantable 100, le système de communication 1 est configuré pour permettre une opération d'écriture du dispositif médical actif implantable 100 du patient depuis son domicile 107 par un dispositif clinicien 200 situé dans centre hospitalier 203 telle que la distance D entre le dispositif médical actif implantable 100 et le dispositif clinicien 200 est de plusieurs kilomètres. Pour ce faire, le système de communication 1 utilise une fonction de télémétrie de longue portée.

Afin de sécuriser la communication par télémétrie de longue portée, le système de communication 1 comprend également un dispositif intermédiaire de proximité sans fil 110 externe et non-implantable. Le dispositif intermédiaire de proximité 110 est configuré pour communiquer par radiofréquence avec le dispositif médical implantable 100 à une distance d₁ du dispositif médical implantable 100, tel que d₁ soit inférieure à 10 mètres. Le dispositif intermédiaire de proximité 110 peut être un smartphone, une tablette numérique, un dispositif portable connecté, un ordinateur, un dispositif d'équipement médical à domicile ou un système domotique. Le dispositif intermédiaire de proximité 110 est donc adapté pour un usage au domicile 107 d'un patient. Le dispositif intermédiaire de proximité 110 est configuré pour acquérir des données transmises par un dispositif clinicien 200, aussi appelé dans ce qui suit dispositif principal d'écriture à distance 200. Le dispositif intermédiaire de proximité 110 est configuré pour acquérir des données 205 transmises par le dispositif principal d'écriture 200 par une connexion réseau 300, en particulier par une connexion Internet avec ou sans fil, ou bien une ligne téléphonique. Le dispositif intermédiaire de proximité 110 comprend un moyen de stockage 113 afin de stocker les données d'écriture 205, au moins jusqu'à l'autorisation d'accéder au dispositif médical implantable 100. De plus, le dispositif intermédiaire de proximité 110 est également configuré pour recevoir des données du dispositif médical actif implantable 100 par radiofréquence et de les transmettre au dispositif principal 200 à distance par une connexion réseau. Ainsi, le système 1 est configuré à la fois pour une opération d'écriture de la mémoire 105 du dispositif médical implantable 100 mais également pour la réception de données provenant du dispositif médical implanté 100 comme par exemple des données physiologiques acquises par le dispositif implanté 100. Ces données peuvent être transmises par le dispositif intermédiaire de proximité 110 au dispositif principal à distance 200 qui peut, par exemple, adapter en retour l'opération d'écriture de la mémoire 105 du dispositif médical implantable 100.

Selon un mode de réalisation préféré, le dispositif principal d'écriture 200 a accès à un site web, sur lequel un clinicien peut entrer les données d'écriture 205 et telle que le dispositif intermédiaire de proximité 110 ait également accès à ce site web via une connexion Internet et peut donc acquérir lesdites données d'écriture 205. Selon un autre mode de réalisation d'autres types de connexion réseau de longue portée peuvent être établis entre le dispositif intermédiaire de proximité 110 et le dispositif principal d'écriture 200. La connexion réseau 300 permet une communication entre le dispositif intermédiaire de proximité 110 et le dispositif principal écriture 200 sur une distance de plusieurs kilomètres, notamment sur la distance D entre le domicile 107 d'un patient et un centre hospitalier 203 où se trouve le dispositif clinicien 200. Ainsi, le dispositif intermédiaire de proximité 110 met en relation le dispositif médical implantable 100 et le dispositif principal écriture 200 sans qu'il n'y ait toutefois de communication directe par connexion réseau entre le dispositif principal d'écriture 200 et le dispositif médical implantable 100 afin de diminuer les risques de piratage du dispositif médical implantable 100.

De plus, afin d'alerter le patient qu'un clinicien souhaite procéder à une opération d'écriture ou de mise à jour du logiciel de son dispositif médical implantable 100, le dispositif intermédiaire de proximité 110 du patient comprend un moyen de signalisation 115 qui émet une notification, par exemple une notification sous la forme d'un message d'alerte et/ou d'un signal sonore et/ou visuel et/ou vibrant.

Pour améliorer davantage la sécurité de la connexion par réseau 300, le système de communication 1 comprend un premier outil de déverrouillage 120 externe non-implantable, distinct du dispositif non-implantable intermédiaire de proximité sans fil 110 qui est configuré pour émettre un signal 121 détectable par le capteur de proximité 103 du dispositif médical implantable 100. Selon le mode de réalisation illustrée à la figure 1, le premier outil de déverrouillage 120 est un aimant permanent ou un électroaimant dont le champ magnétique statique est détecté par le capteur de proximité 103 du dispositif médical actif implantable 100. Dans un autre mode de réalisation, le premier outil de déverrouillage 120 est configuré pour émettre un signal 121 provenant d'un champ électromagnétique, d'un champ inductif ou d'un système de communication de corps humain. Lorsque le premier outil de déverrouillage 120 est localisé dans un périmètre prédéterminé, c'est-à-dire à moins d'une distance d₂ du dispositif médical 100 ; telle que la distance d₂ soit inférieure à 15 centimètres, en particulier inférieure à 10 centimètres ; la présence du premier outil de déverrouillage 120 est détectée par le dispositif médical implantable 100. La détection de la présence du premier outil de déverrouillage 120 dans le périmètre prédéterminé autorise l'accès du dispositif médical actif implantable 100 au dispositif intermédiaire de proximité 110. En conséquence, la communication par radiofréquence 130 entre le dispositif médical actif implantable 100 au dispositif intermédiaire de proximité 110 est autorisée. L'autorisation de la communication par radiofréquence 130 nécessite donc qu'un patient positionne le premier outil de déverrouillage 120 suffisamment près, c'est-à-dire à moins de 15 cm, de son dispositif médical implantable 100 afin de le déverrouiller.

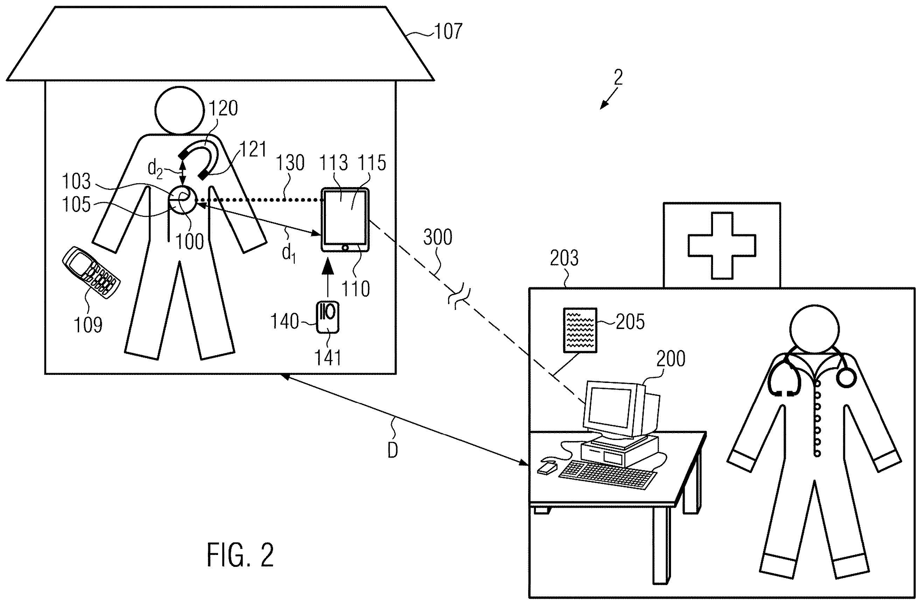

La Figure 2 illustre schématiquement un système de communication 2 pour permettre une opération d'écriture d'un dispositif médical actif implantable 100 selon un deuxième mode de réalisation de l'invention. Les éléments avec les mêmes références numériques déjà utilisées pour la description de la Figure 1 ne seront pas décrits à nouveau en détail, et référence est faite à leurs descriptions ci-dessus.

Le système de communication 2 comprend le dispositif implantable médicale actif 100, le dispositif intermédiaire de proximité 110, le dispositif principal d'écriture 200 et le premier outil de déverrouillage 120 tels que décrits ci-dessus pour le système de communication 1.

Afin d'améliorer davantage la sécurité de l'opération d'écriture par télémétrie de longue portée, le système de communication 2 comprend également un deuxième outil de déverrouillage 140 configuré pour déverrouiller le dispositif intermédiaire de proximité 110 et ainsi pour autoriser la communication par radiofréquence entre le dispositif médical actif implantable 100 et le dispositif intermédiaire de proximité 110. Selon le deuxième mode de réalisation illustré par la Figure 2, le deuxième outil de déverrouillage 140 est une carte magnétique 141 configurée pour être scannée et lue par le dispositif intermédiaire de proximité 110. Selon d'autres modes de réalisation, le deuxième outil de déverrouillage 140 peut aussi au moins comprendre un mot de passe à entrer dans le dispositif intermédiaire de proximité 110, une entrée biométrique reçue par le dispositif intermédiaire de proximité 110, une communication sans fil de courte distance qui émet un signal détectable par un capteur de proximité du dispositif intermédiaire de proximité 110, un champ magnétique détecté par le dispositif intermédiaire de proximité 110 ou une transmission reçue par l'intermédiaire d'un port périphérique du dispositif intermédiaire de proximité 110, comme un port USB par exemple.

Ainsi, selon le deuxième de mode de réalisation, l'autorisation de réécriture dans la mémoire 105 du dispositif médical actif implantable 100 dans le système 2 nécessite qu'un patient positionne le premier outil de déverrouillage 120 suffisamment près, c'est-à-dire à moins de 15 cm, de son dispositif médical implantable 100 afin de le déverrouiller, et qu'il scanne une carte magnétique personnelle 141 afin qu'elle soit lue et identifiée par le dispositif intermédiaire de proximité 110. De ce fait, dans le deuxième mode de réalisation, le système 2 comprend deux moyens de déverrouillage de proximité 120, 140 qui sont configurés pour être opérés à proximité immédiate du patient (moins de 15 centimètre pour le premier outil 120) et/ou par le patient lui-même. En conséquence, ces outils de proximité de déverrouillage 120, 140 permettent d'améliorer et de sécuriser une opération d'écriture dans la mémoire 105 du dispositif médical implantable 100 par télémétrie de longue portée.

Cette opération d'écriture est signalée au patient par le moyen de signalisation 115 du dispositif intermédiaire de proximité 110 qui émet une notification, par exemple une notification sous la forme d'un message d'alerte et/ou d'un signal sonore et/ou visuel et/ou vibrant. De plus, selon le deuxième mode de réalisation, une notification peut aussi être envoyée en parallèle à un dispositif portable du patient 109 tel que son téléphone mobile 109 ou une montre connectée, afin de s'assurer qu'il soit informé de la requête pour l'opération d'écriture, et ce, même lorsque qu'il n'a pas le dispositif intermédiaire de proximité 110 directement à portée de main. La notification peut être au moins un signal visuel, un signal sonore, une vibration, un message écrit, un message vocal au moins sur le dispositif intermédiaire de proximité 110. Le dispositif portable 109 du patient peut recevoir en parallèle une notification de même catégorie ou différente de celle envoyée sur le dispositif intermédiaire de proximité 110.

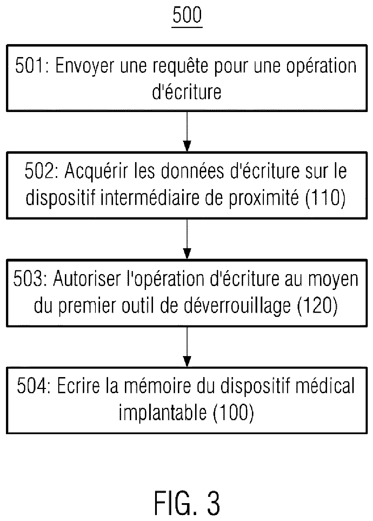

Le fonctionnement du système de communication 1 va être expliqué dans ce qui suit via la description de l'organigramme 500 de la Figure 3. Les éléments avec les mêmes références numériques déjà utilisées pour la description de la Figure 1 ne seront pas décrits à nouveau en détail, et référence est faite à leurs descriptions ci-dessus.

Lorsqu'une mise à jour, une réécriture de la mémoire 105, un ajustement ou une reprogrammation du logiciel du dispositif médical implantable 100 du patient est nécessaire, un clinicien entre une requête dans le dispositif clinicien 200 afin de notifier le patient d'une demande d'opération d'écriture. De ce fait, à la première étape 501 de la méthode 500 illustrée par la Figure 3, une requête est envoyée depuis le dispositif clinicien 200 par connexion réseau, en particulier par une connexion Internet, au dispositif intermédiaire de proximité 110 du patient. La réception de cette requête au niveau du dispositif intermédiaire de proximité 110 est signalée au patient par le moyen de signalisation 115 du dispositif intermédiaire de proximité 110 qui émet une notification sonore, d'un clignotant lumineux, d'un message et/ou d'une vibration.

La première étape 501 est suivie par l'étape 502 d'acquisition des données d'écriture 205 par le dispositif intermédiaire de proximité 110. Les données d'écriture 205 sont entrées dans le dispositif principal d'écriture 200 par un clinicien. Ces données d'écriture 205 sont transmises par télémétrie de longue portée au dispositif intermédiaire de proximité 110. En particulier, ces données d'écriture 205 sont entrées sur un site web et transmises via une connexion Internet au dispositif intermédiaire de proximité 110 qui a lui aussi accès à ce site web afin d'acquérir les données d'écriture 205. Le dispositif intermédiaire de proximité 110 comprend un moyen de stockage 113 afin de stocker les données d'écriture 205, au moins jusqu'à l'autorisation d'accéder au dispositif médical implantable 100, c'est-à-dire jusqu'à ce que la réécriture dans la mémoire 105 du dispositif médical implantable 100 ait été autorisée par la détection du premier outil de déverrouillage 120.

En effet, l'opération d'écriture de la mémoire 105 du dispositif médical implantable 100 nécessite d'autoriser le dispositif intermédiaire de proximité 110 à transmettre et recevoir des données au dispositif médical implantable 100.

C'est pourquoi lors d'une deuxième étape 503 de la méthode 500 illustrée par la Figure 3, le premier outil de déverrouillage 120 est demandé à être placé dans le périmètre de proximité prédéterminé du dispositif médical actif implantable 100, c'est-à-dire à environ moins de 10 centimètres, afin que le capteur de proximité 103 du dispositif médical implantable 100 détecte la présence du premier outil de verrouillage 120. La détection du premier outil de déverrouillage 120 permet d'autoriser une communication par radiofréquence 130 entre le dispositif intermédiaire de proximité 110 et le dispositif médical actif implantable 100. La non-détection du premier outil de déverrouillage 120 par le capteur de proximité du dispositif médical implantable 130 empêche une opération d'écriture de la mémoire 105 du dispositif médical implantable 100.

Si les conditions de déverrouillage sont validées à l'étape 503, l'accès au dispositif médical implantable 100 est autorisé pour le dispositif intermédiaire de proximité 110 pour un laps de temps prédéterminé. Après ce laps de temps prédéterminé écoulé, l'accès au dispositif médical implantable 100 est verrouillé tel que le dispositif intermédiaire de proximité 110 ou tout autre dispositif de télémétrie ne puisse plus accéder au dispositif médical implantable 100. Selon un mode de réalisation avantageux, le laps de temps prédéterminé commence à s'écouler dès la réception par le dispositif intermédiaire de proximité 110 de la requête pour l'opération d'écriture, c'est-à-dire à l'étape 501.

À l'étape 504 de la méthode 500, les données d'écriture 205 acquises par le dispositif intermédiaire 110 sont transmises au dispositif médical actif implantable 100. Il en résulte une opération d'écriture dans la mémoire 105 à distance du dispositif médical implantable 100 selon les données 205 entrées par le clinicien.

Lorsque l'écriture dans la mémoire 105 du dispositif médical implantable 100 ou toutes autres opérations de reprogrammation, de mise à jour ou d'ajustement du dispositif médical implantable 100, est réalisée avec succès, le dispositif médical implantable 100 envoie une notification de fin de processus au dispositif intermédiaire de proximité 110 qui informe le patient qu'il peut retirer le premier outil de déverrouillage 120 et qui transmet la notification de fin de processus au dispositif clinicien 200. Le dispositif clinicien 200, et donc le clinicien lui-même, est ainsi informé de la réussite ou de l'échec de l'opération de réécriture. Selon un mode de réalisation avantageux, une durée de temps prédéterminé doit être respectée entre chaque tentative d'opération de réécriture du dispositif médical implantable 100.

Ainsi, la méthode 500 permet d'améliorer la sécurité de la communication à distance avec le dispositif médical actif implantable 100 grâce à une première barrière de sécurité établie par le dispositif intermédiaire de proximité 110 qui empêche un accès direct du dispositif d'écriture 200 au dispositif médical implantable 100 par connexion réseau ; ainsi qu'une seconde barrière de sécurité grâce au premier outil de déverrouillage de proximité 120 à courte portée qui autorise l'accès au dispositif médical implanté 100 par l'intermédiaire du dispositif intermédiaire de proximité 110. Ainsi, l'opération d'écriture ne peut se faire que sur la requête du clinicien et l'acceptation de cette requête par le patient qui doit alors autoriser l'accès à son dispositif médical implantable 100 par l'usage du premier outil de déverrouillage 120 dans le voisinage immédiat de son dispositif médical implantable 100. La succession de ces étapes permet d'améliorer davantage la sûreté des échanges par connexion réseau.

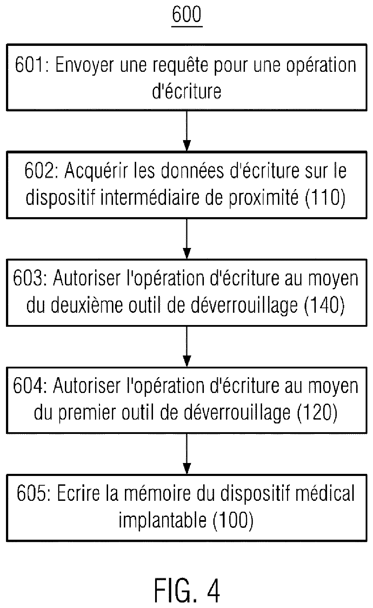

Le fonctionnement du système de communication 2 va être expliqué dans ce qui suit via la description de l'organigramme 600 de la Figure 4. Les éléments avec les mêmes références numériques déjà utilisées pour la description des Figures 1, 2 et 3 ne seront pas décrits à nouveau en détail, et référence est faite à leurs descriptions ci-dessus.

Selon la méthode du deuxième mode de réalisation, lorsqu'une mise à jour, une réécriture de la mémoire 105, un ajustement ou une reprogrammation du logiciel du dispositif médical implantable 100 du patient est nécessaire, un clinicien entre une requête dans le dispositif clinicien 200 afin de notifier le patient d'une demande d'opération d'écriture. De ce fait, à la première étape 601 de la méthode 600 illustrée par la Figure 4, une requête est envoyée depuis le dispositif clinicien par connexion réseau, en particulier par une connexion Internet, au dispositif intermédiaire de proximité 110 du patient. La réception de cette requête au niveau du dispositif intermédiaire de proximité 110 est signalée au patient par le moyen de signalisation 115 du dispositif intermédiaire de proximité 110 qui émet une notification sonore, d'un clignotant lumineux, d'un message et/ou d'une vibration. En parallèle, selon le deuxième mode de réalisation, une notification, par exemple un message, un SMS (pour « Short Message Service » en anglais), un email ou autre, peut également être envoyée à un dispositif portable du patient 109 tel que sur son téléphone portable 109 ou sa montre connectée. La notification indique au patient la requête d'une opération de réécriture par télémétrie de longue portée. L'opération d'écriture de la mémoire 105 du dispositif médical implantable 100 nécessite d'autoriser le dispositif intermédiaire de proximité 110 à transmettre et recevoir des données au dispositif médical implantable 100.

La première étape 601 est suivie par l'étape 602 d'acquisition des données d'écriture 205 par le dispositif intermédiaire de proximité 110. Les données d'écriture 205 sont entrées dans le dispositif principal d'écriture 200 par un clinicien. Ces données d'écriture 205 sont transmises par télémétrie de longue portée au dispositif intermédiaire de proximité 110. En particulier, ces données d'écriture 205 sont entrées sur un site web et transmises via une connexion Internet au dispositif intermédiaire de proximité 110 qui a lui aussi accès à ce site web afin d'acquérir les données d'écriture 205. Les données d'écriture 205 sont stockées par les moyen de stockage 113 du dispositif intermédiaire 110 au moins jusqu'à l'autorisation d'accéder au dispositif médical implantable 100, c'est-à-dire jusqu'à ce que la réécriture dans la mémoire 105 du dispositif médical implantable 100 soit autorisée par la détection des outils de déverrouillage 120 et 140. En effet, l'opération d'écriture de la mémoire 105 du dispositif médical implantable 100 nécessite d'autoriser le dispositif intermédiaire de proximité 110 à transmettre et recevoir des données au dispositif médical implantable 100.

C'est pourquoi, lors d'une troisième étape 603 de la méthode 600, l'autorisation de la communication par radiofréquence 130 entre le dispositif intermédiaire de proximité 110 et le dispositif médical actif implantable 100 doit être autorisée au moyen du deuxième outil de déverrouillage externe non-implantable 140. La non-validation du deuxième outil de déverrouillage 140 par le dispositif actif médical implantable 100 empêche une opération d'écriture dans la mémoire 105 du dispositif médical implantable 100.

Puis, lors d'une quatrième étape 604 de la méthode 600 illustrée par la Figure 4, le premier outil de déverrouillage 120 est demandé à être placé dans le périmètre de proximité prédéterminé du dispositif médical actif implantable 100, c'est-à-dire à environ moins de 10 centimètres, afin que le capteur de proximité 103 du dispositif médical implantable 100 détecte la présence du premier outil de verrouillage 103. La détection du premier outil de déverrouillage 103 permet d'autoriser une communication par radiofréquence 130 entre le dispositif intermédiaire de proximité 110 et le dispositif médical actif implantable 100. La non-détection du premier outil de déverrouillage 120 par le capteur de proximité du dispositif médical implantable 130 empêche une opération d'écriture de la mémoire 105 du dispositif médical implantable 100.

Dans une variante qui n'est pas illustrée par la Figure 4, l'étape 603 peut être réalisée après l'étape 604. Dans une autre variante, les étapes 603 et 604 peuvent être réalisées simultanément.

Si les conditions de déverrouillage sont validées aux étapes 603 et 604, l'accès au dispositif médical implantable 100 est autorisé pour le dispositif intermédiaire de proximité 110 pour un laps de temps prédéterminé. Après ce laps de temps prédéterminé écoulé, l'accès au dispositif médical implantable 100 est verrouillé tel que le dispositif intermédiaire de proximité 110 ou tout autre dispositif de télémétrie ne puisse plus accéder au dispositif médical implantable 100. Selon un mode de réalisation avantageux, le laps de temps prédéterminé commence à s'écouler dès la réception par le dispositif intermédiaire de proximité 110 de la requête pour l'opération d'écriture, c'est-à-dire à l'étape 601.

À l'étape 605 de la méthode 600, les données d'écriture 205 acquises par le dispositif intermédiaire de proximité 110 sont transmises au dispositif médical actif implantable 100. Il en résulte une opération d'écriture dans la mémoire 105 à distance du dispositif médical implantable 100 selon les données 205 entrées par le clinicien.

Lorsque l'écriture dans la mémoire 105 du dispositif médical implantable 100 ou toutes autres opérations de reprogrammation, de mise à jour ou d'ajustement du dispositif médical implantable 100, est réalisée avec succès, le dispositif médical implantable 100 envoie une notification de fin de processus au dispositif intermédiaire de proximité 110 qui informe le patient qu'il peut retirer les outils de verrouillage 120 et 140 et qui transmet la notification de fin de processus au dispositif clinicien 200. Le dispositif clinicien 200, et donc le clinicien lui-même, est ainsi informé de la réussite ou de l'échec de l'opération de réécriture. Selon un mode de réalisation avantageux, une durée de temps prédéterminé doit être respectée entre chaque tentative d'opération de réécriture du dispositif médical implantable 100.

Ainsi, la méthode 600 permet d'améliorer la sécurité de la communication à distance avec le dispositif médical actif implantable 100 grâce à une première barrière de sécurité établie par le dispositif intermédiaire de proximité 110 qui empêche un accès direct du dispositif d'écriture 200 au dispositif médical implantable 100 par connexion réseau ; ainsi qu'une seconde barrière de sécurité grâce au premier outil de déverrouillage 120 à courte portée et au deuxième outil de déverrouillage 140 qui autorisent l'accès au dispositif médical implanté 100 par l'intermédiaire du dispositif intermédiaire de proximité 110. Ainsi, l'opération d'écriture ne peut se faire que sur la requête du clinicien et l'acceptation de cette requête par le patient qui doit alors autoriser l'accès à son dispositif médical implantable 100 par l'usage du premier 120 et du deuxième outil de déverrouillage140. La succession de ces étapes permette d'améliorer davantage la sûreté des échanges par connexion réseau.

Les systèmes 1 et 2 et les méthodes 500 et 600 de la présente invention permettent ainsi l'amélioration de la sécurité de la télémétrie de longue portée par connexion réseau et ainsi d'optimiser l'écriture dans la mémoire 105 du dispositif médical implanté 100 par un dispositif d'écriture 200 d'un clinicien qui se trouve éloigné de plusieurs kilomètres du domicile du patient implanté.

Les modes de réalisation décrits sont simplement des configurations possibles et il faut garder à l'esprit que les caractéristiques individuelles des différents modes de réalisation peuvent être combinées entre elles ou fournies indépendamment les unes des autres.

## Revendications

1. Un système de communication (2) pour permettre une opération d'écriture dans la mémoire d'un dispositif médical actif implantable, le système (2) comprenant :
ledit dispositif médical actif implantable (100) qui comprend au moins un capteur de proximité (103) ;
un dispositif principal d'écriture à distance non implantable (200) ;
un dispositif intermédiaire de proximité (110) externe non implantable qui est configuré pour recevoir des instructions et données d'écriture (205) transmises par le dispositif principal d'écriture à distance (200) par une connexion réseau (300), le dispositif intermédiaire de proximité (110) étant configuré pour communiquer sans fil avec le dispositif médical implantable (100) ;
un premier outil de déverrouillage (120) externe non implantable, distinct du dispositif intermédiaire de proximité (110), qui est configuré pour émettre un signal détectable par le capteur de proximité (103) lorsque le premier outil de déverrouillage (120) est localisé dans un périmètre prédéterminé du dispositif médical (100),
dans lequel le dispositif intermédiaire de proximité (110) est configuré pour écrire dans la mémoire (105) du dispositif médical actif implantable (100) selon les instructions (205) transmises par le dispositif d'écriture principal à distance (200) en réponse à une détection de la présence dans le périmètre prédéterminé du premier outil de déverrouillage (120) par le capteur de proximité (103) du dispositif médical actif implantable (100),
dans lequel le premier outil de déverrouillage (120) est un aimant permanent ou un électroaimant dont le champ magnétique statique est détecté par le capteur de proximité (103) du dispositif médical actif implantable (100),
**caractérisé en ce que** le système (2) comprend en outre :
un deuxième outil de déverrouillage (140) configuré pour autoriser la communication par radiofréquence (130) entre le dispositif médical actif implantable (100) et le dispositif intermédiaire de proximité (110) lorsque le dispositif intermédiaire de proximité (110) a identifié le deuxième outil de déverrouillage (140), de sorte que le dispositif intermédiaire de proximité (110) est configuré pour écrire dans la mémoire (105) du dispositif médical actif implantable (100) selon les instructions (205) transmises par le dispositif d'écriture principal à distance (200) en réponse à l'identification du deuxième outil de déverrouillage (140) par le dispositif intermédiaire de proximité (110),
dans lequel le deuxième outil de déverrouillage (140) comprend au moins un mot de passe entré sur le dispositif intermédiaire de proximité (110), une entrée biométrique reçue par le dispositif intermédiaire de proximité (110), une carte magnétique (141) lue par le dispositif intermédiaire de proximité (110), une communication sans fil de courte distance qui émet un signal détectable par un capteur de proximité (103) du dispositif intermédiaire de proximité (110), un champ magnétique détecté par le dispositif intermédiaire de proximité (110) ou une transmission reçue par l'intermédiaire d'un port périphérique du dispositif intermédiaire de proximité (110), en particulier un port USB.

2. Le système selon la revendication 1, dans lequel le dispositif intermédiaire de proximité (110) comprend un moyen de signalisation (115) pour fournir un signal visuel et/ou sonore et/ou vibrant indiquant qu'une opération d'écriture doit être effectuée.

3. Le système selon l'une des revendications précédentes, dans lequel le dispositif intermédiaire de proximité (110) comprend au moins un moyen de stockage (113) pour stocker les données d'écriture (205) transmises par le dispositif d'écriture principal à distance (200) au moins jusqu'à ce que l'opération d'écriture soit activée.

4. Le système selon l'une des revendications précédentes, dont le dispositif intermédiaire de proximité non implantable (110) est un smartphone, une tablette numérique, un dispositif portable connecté, un ordinateur, un dispositif d'équipement médical à domicile ou un système domotique.

5. Le système selon l'une des revendications précédentes, comprenant en outre un dispositif de communication portatif (109), en particulier à un téléphone portable (109), une tablette, un ordinateur portable et/ou une montre connectée ; auquel la requête pour une opération d'écriture est envoyée par connexion réseau via un message, un message de Short Message Service, SMS, un email ou un type d'alerte analogue.

6. Méthode (600) pour permettre une opération d'écriture dans la mémoire d'un dispositif médical actif implantable, la méthode (600) comprenant les étapes de :
a) envoyer (601) à un dispositif non implantable intermédiaire de proximité sans fil (110) une requête par connexion réseau (300) pour une opération d'écriture du dispositif médical actif implantable (100) par un dispositif d'écriture principal à distance (200) ;
b) recevoir (602) par le dispositif intermédiaire de proximité (110) des instructions et des données d'écriture (205) envoyées par connexion réseau (300) par le dispositif d'écriture principal à distance (200) ;
c) placer un premier outil de déverrouillage externe non implantable (120) dans un périmètre de proximité prédéterminé du dispositif médical actif implantable (100) afin d'autoriser (604) une communication par radiofréquence (130) entre le dispositif intermédiaire de proximité (110) et le dispositif médical actif implantable (100),
dans laquelle le premier outil de déverrouillage (120) émet un signal au dispositif médical actif implantable (100) qui provient d'un champ électromagnétique ou d'un champ magnétique statique ou d'un champ inductif ;
d) écrire (605) lesdites données d'écriture (205) dans la mémoire (105) du dispositif médical actif implantable (100) par le dispositif intermédiaire de proximité (110),
la méthode (600) étant **caractérisée en ce que** :
l'étape c) est précédée d'une étape pour recevoir, via le dispositif intermédiaire de proximité (110), la requête d'autoriser (603) la communication par radiofréquence (130) entre le dispositif intermédiaire de proximité (110) et le dispositif médical actif implantable (100) au moyen d'un deuxième outil de déverrouillage externe non implantable (140),
dans laquelle le deuxième outil de déverrouillage (140) comprend au moins un mot de passe entré sur le dispositif intermédiaire de proximité (110), une entrée biométrique reçue le dispositif intermédiaire de proximité (110), une carte magnétique (141) lue par le dispositif intermédiaire de proximité (110), une communication sans fil de courte distance qui émet un signal détectable par un capteur de proximité du dispositif intermédiaire de proximité (110), un champ magnétique détecté par le dispositif intermédiaire de proximité (110) ou une transmission reçue par l'intermédiaire d'un port périphérique du dispositif intermédiaire de proximité (110), en particulier un port USB.

7. La méthode selon la revendication 6, dans laquelle l'opération d'écriture du dispositif médical actif implantable (100) est empêchée lorsque le premier outil de déverrouillage (120) n'est pas détecté par le dispositif médical actif implantable (100), en particulier lorsque la distance (d2) entre le premier outil de déverrouillage (120) et le dispositif médical actif implantable (100) est plus grande que 15 centimètres, plus en particulier plus grande que 10 centimètres.

8. La méthode selon l'une des revendications 6 à 7, dans laquelle la communication par radiofréquence (130) entre le dispositif médical actif implantable (100) et le dispositif de communication intermédiaire (110) est réalisée lorsque lesdits dispositifs (110, 130) sont distants l'un de l'autre de moins de 10 mètres.

9. La méthode selon l'une des revendications 6 à 8, dans laquelle, à l'étape a), la requête pour une opération d'écriture est signalée par un moyen de signalisation (115) du dispositif intermédiaire de proximité (110), qui émet un signal visuel et/ou sonore et/ou vibrant indiquant qu'une opération d'écriture doit être effectuée.

10. La méthode selon l'une des revendications 6 à 9, dans laquelle, à l'étape a), la requête pour une opération d'écriture est également envoyée par connexion réseau à un dispositif de communication portatif (109), en particulier à un téléphone portable, une tablette, un ordinateur portable et/ou une montre connectée via un message, un message de Short Message Service, SMS, un email ou un type d'alerte analogue.

11. La méthode selon l'une des revendications 6 à 10, dans laquelle les données d'écriture (205) sont stockées par un moyen de stockage (113) du dispositif intermédiaire de proximité (110) au moins jusqu'à l'autorisation d'une communication par radiofréquence (130) entre le dispositif intermédiaire de proximité (110) et le dispositif médical actif implantable (100).

## Patentansprüche

1. Ein Kommunikationssystem (2) zur Ermöglichung eines Schreibvorgangs in einem Speicher eines implantierbaren aktiven medizinischen Geräts, wobei das System (2) umfasst:
das implantierbare aktive medizinische Gerät (100), das mindestens einen Näherungssensor (103) umfasst;
ein nicht implantierbares entferntes Hauptschreibgerät (200);
ein externes nicht implantierbares intermediäres Näherungsgerät (110), das konfiguriert ist, Schreibanweisungen und Daten (205), die vom entfernten Hauptschreibgerät (200) über eine Netzwerkverbindung (300) übertragen werden, zu empfangen, wobei das intermediäre Näherungsgerät (110) konfiguriert ist, drahtlos mit dem implantierbaren medizinischen Gerät (100) zu kommunizieren;
ein erstes nicht implantierbares externes Freigabewerkzeug (120), das sich vom intermediären Näherungsgerät (110) unterscheidet und konfiguriert ist, ein vom Näherungssensor (103) detektierbares Signal auszusenden, wenn sich das erste Freigabewerkzeug (120) innerhalb eines vorbestimmten Umkreises des medizinischen Geräts (100) befindet,
wobei das intermediäre Näherungsgerät (110) konfiguriert ist, gemäß den vom entfernten Hauptschreibgerät (200) übertragenen Anweisungen (205) in den Speicher (105) des implantierbaren aktiven medizinischen Geräts (100) zu schreiben, als Reaktion auf die Detektion der Anwesenheit des ersten Freigabewerkzeugs (120) innerhalb des vorbestimmten Umkreises durch den Näherungssensor (103) des aktiven implantierbaren medizinischen Geräts (100),
wobei das erste Freigabewerkzeug (120) ein Dauermagnet oder ein Elektromagnet ist, dessen statisches Magnetfeld vom Näherungssensor (103) des implantierbaren aktiven medizinischen Geräts (100) detektiert wird,
**dadurch gekennzeichnet, dass** das System (2) ferner umfasst:
ein zweites Freigabewerkzeug (140), das konfiguriert ist, eine Hochfrequenzkommunikation (130) zwischen dem implantierbaren aktiven medizinischen Gerät (100) und dem intermediären Näherungsgerät (110) zu ermöglichen, wenn das intermediäre Näherungsgerät (110) das zweite Freigabewerkzeug (140) identifiziert hat, sodass das intermediäre Näherungsgerät (110) konfiguriert ist, gemäß den vom entfernten Hauptschreibgerät (200) übertragenen Anweisungen (205) in den Speicher (105) des implantierbaren aktiven medizinischen Geräts (100) zu schreiben, als Reaktion auf die genannte Identifikation des zweiten Freigabewerkzeugs (140) durch das intermediäre Näherungsgerät (110),
wobei das zweite Freigabewerkzeug (140) mindestens eines der folgenden umfasst: ein auf dem intermediären Näherungsgerät (110) eingegebenes Passwort, eine vom intermediären Näherungsgerät (110) empfangene biometrische Eingabe, eine vom intermediären Näherungsgerät (110) gelesene Magnetkarte (141), eine Nahbereichsfunkkommunikation, die ein vom Näherungssensor (103) des intermediären Näherungsgeräts (110) detektierbares Signal aussendet, ein vom intermediären Näherungsgerät (110) detektiertes Magnetfeld oder eine über einen Peripherieanschluss des intermediären Näherungsgeräts (110), insbesondere einen USB-Anschluss, empfangene Übertragung.

2. System nach Anspruch 1, wobei das intermediäre Näherungsgerät (110) ein Signalmittel (115) umfasst, das ein visuelles und/oder akustisches und/oder vibrierendes Signal zur Anzeige eines auszuführenden Schreibvorgangs bereitstellt.

3. System nach einem der vorhergehenden Ansprüche, wobei das intermediäre Näherungsgerät (110) mindestens ein Speichermittel (113) umfasst, um die vom entfernten Hauptschreibgerät (200) übertragenen Schreibdaten (205) mindestens bis zur Aktivierung des Schreibvorgangs zu speichern.

4. System nach einem der vorhergehenden Ansprüche, wobei das nicht implantierbare intermediäre Näherungsgerät (110) ein Smartphone, ein digitales Tablet, ein verbundenes tragbares Gerät, ein Computer, ein medizinisches Heimgerät oder ein Hausautomationssystem ist.

5. System nach einem der vorhergehenden Ansprüche, ferner umfassend ein tragbares Kommunikationsgerät (109), insbesondere ein Mobiltelefon (109), ein Tablet, einen Laptop und/oder eine Smartwatch, an das die Anforderung eines Schreibvorgangs über eine Netzwerkverbindung mittels einer Nachricht, einer SMS (Short Message Service), einer E-Mail oder einer ähnlichen Art von Benachrichtigung gesendet wird.

6. Verfahren (600) zur Ermöglichung eines Schreibvorgangs in einem Speicher eines implantierbaren aktiven medizinischen Geräts, wobei das Verfahren (600) die folgenden Schritte umfasst:
a) Senden (601) einer Anforderung über eine Netzwerkverbindung (300) an ein drahtloses nicht implantierbares intermediäres Näherungsgerät (110) für einen Schreibvorgang des implantierbaren aktiven medizinischen Geräts (100) durch ein entferntes Hauptschreibgerät (200);
b) Empfangen (602) von Schreibanweisungen und Daten (205) durch das intermediäre Näherungsgerät (110), die über eine Netzwerkverbindung (300) vom entfernten Hauptschreibgerät (200) gesendet werden;
c) Platzieren eines ersten nicht implantierbaren externen Freigabewerkzeugs (120) innerhalb eines vorbestimmten Näherungsumkreises des implantierbaren aktiven medizinischen Geräts (100), um eine Hochfrequenzkommunikation (130) zwischen dem intermediären Näherungsgerät (110) und dem implantierbaren aktiven medizinischen Gerät (100) zu ermöglichen (604),
wobei das erste Freigabewerkzeug (120) ein Signal an das implantierbare aktive medizinische Gerät (100) aussendet, das von einem elektromagnetischen Feld, einem statischen Magnetfeld oder einem induktiven Feld stammt;
d) Schreiben (605) der genannten Schreibdaten (205) in den Speicher (105) des implantierbaren aktiven medizinischen Geräts (100) durch das intermediäre Näherungsgerät (110),
wobei das Verfahren (600) **dadurch gekennzeichnet ist, dass**:
Schritt c) durch einen Schritt des Empfangens, über das intermediäre Näherungsgerät (110), der Anforderung zur Ermöglichung (603) der Hochfrequenzkommunikation (130) zwischen dem intermediären Näherungsgerät (110) und dem implantierbaren aktiven medizinischen Gerät (100) mittels eines zweiten nicht implantierbaren externen Freigabewerkzeugs (140) vorangeht,
wobei das zweite Freigabewerkzeug (140) mindestens eines der folgenden umfasst: ein auf dem intermediären Näherungsgerät (110) eingegebenes Passwort, eine vom intermediären Näherungsgerät (110) empfangene biometrische Eingabe, eine vom intermediären Näherungsgerät (110) gelesene Magnetkarte (141), eine Nahbereichsfunkkommunikation, die ein vom Näherungssensor des intermediären Näherungsgeräts (110) detektierbares Signal aussendet, ein vom intermediären Näherungsgerät (110) detektiertes Magnetfeld oder eine über einen Peripherieanschluss des intermediären Näherungsgeräts (110), insbesondere einen USB-Anschluss, empfangene Übertragung.

7. Verfahren nach Anspruch 6, wobei der Schreibvorgang des implantierbaren aktiven medizinischen Geräts (100) verhindert wird, wenn das erste Freigabewerkzeug (120) vom implantierbaren aktiven medizinischen Gerät (100) nicht detektiert wird, insbesondere wenn der Abstand (d2) zwischen dem ersten Freigabewerkzeug (120) und dem implantierbaren aktiven medizinischen Gerät (100) größer als 15 Zentimeter, insbesondere größer als 10 Zentimeter ist.

8. Verfahren nach einem der Ansprüche 6 bis 7, wobei die Hochfrequenzkommunikation (130) zwischen dem implantierbaren aktiven medizinischen Gerät (100) und dem intermediären Näherungsgerät (110) durchgeführt wird, wenn die genannten Geräte (100, 110) durch eine Entfernung von weniger als 10 Metern getrennt sind.

9. Verfahren nach einem der Ansprüche 6 bis 8, wobei in Schritt a) die Anforderung eines Schreibvorgangs durch ein Signalmittel (115) des intermediären Näherungsgeräts (110) signalisiert wird, das ein visuelles und/oder akustisches und/oder vibrierendes Signal aussendet, das anzeigt, dass ein Schreibvorgang auszuführen ist.

10. Verfahren nach einem der Ansprüche 6 bis 9, wobei in Schritt a) die Anforderung eines Schreibvorgangs auch über eine Netzwerkverbindung an ein tragbares Kommunikationsgerät (109), insbesondere ein Mobiltelefon, ein Tablet, einen Laptop und/oder eine Smartwatch, mittels einer Nachricht, einer SMS (Short Message Service), einer E-Mail oder einer ähnlichen Art von Benachrichtigung gesendet wird.

11. Verfahren nach einem der Ansprüche 6 bis 10, wobei die Schreibdaten (205) durch ein Speichermittel (113) des intermediären Näherungsgeräts (110) gespeichert werden, mindestens bis eine Hochfrequenzkommunikation (130) zwischen dem intermediären Näherungsgerät (110) und dem implantierbaren aktiven medizinischen Gerät (100) ermöglicht wird.

## Claims

1. A communication system (2) for enabling a writing operation in a memory of an implantable active medical device, the system (2) comprising:
said implantable active medical device (100), which comprises at least one proximity sensor (103);
a non-implantable remote main writing device (200);
an external non-implantable intermediate proximity device (110), which is configured to receive writing instructions and data (205) transmitted by the remote main writing device (200) via a network connection (300), the intermediate proximity device (110) being configured to wirelessly communicate with the implantable medical device (100);
a first non-implantable external unlocking tool (120), distinct from the intermediate proximity device (110) and configured to emit a signal detectable by the proximity sensor (103) when the first unlocking tool (120) is located within a predetermined perimeter of the medical device (100),
wherein the intermediate proximity device (110) is configured to write into the memory (105) of the implantable active medical device (100) according to the instructions (205) transmitted by the remote main writing device (200) in response to a detection of the presence of the first unlocking tool (120) within the predetermined perimeter by the proximity sensor (103) of the active implantable medical device (100),
wherein the first unlocking tool (120) is a permanent magnet or an electromagnet whose static magnetic field is detected by the proximity sensor (103) of the implantable active medical device (100),
**characterized in that** the system (2) further comprises:
a second unlocking tool (140) configured to allow a radiofrequency communication (130) between the implantable active medical device (100) and the intermediate proximity device (110) when the intermediate proximity device (110) has identified the second unlocking tool (140), so that the intermediate proximity device (110) is configured to write into the memory (105) of the implantable active medical device (100) according to the instructions (205) transmitted by the remote main writing device (200) in response to said identification of the second unlocking tool (140) by the intermediate proximity device (110),
wherein the second unlocking tool (140) comprises at least one of a password entered on the intermediate proximity device (110), a biometric input received by the intermediate proximity device (110), a magnetic card (141) read by the intermediate proximity device (110), a short-range wireless communication emitting a signal detectable by a proximity sensor (103) of the intermediate proximity device (110), a magnetic field detected by the intermediate proximity device (110), or a transmission received via a peripheral port of the intermediate proximity device (110), in particular a USB port.

2. The system of claim 1, wherein the intermediate proximity device (110) comprises a signaling means (115) for providing a visual and/or audible and/or vibrating signal indicating that a writing operation is to be performed.

3. The system of any one of the preceding claims, wherein the intermediate proximity device (110) comprises at least one storage means (113) for storing the writing data (205) transmitted by the remote main writing device (200) at least until the writing operation is activated.

4. The system of any one of the preceding claims, wherein the non-implantable intermediate proximity device (110) is a smartphone, a digital tablet, a connected portable device, a computer, a home medical equipment device, or a home automation system.

5. The system of any one of the preceding claims, further comprising a portable communication device (109), in particular a mobile phone (109), a tablet, a laptop, and/or a smartwatch, to which the request for a writing operation is sent, via a network connection, by means of a message, an SMS (Short Message Service), an email, or a similar type of alert.

6. A method (600) for enabling a writing operation in a memory of an implantable active medical device, the method (600) comprising the steps of:
a) sending (601), to a wireless non-implantable intermediate proximity device (110), a request via a network connection (300) for a writing operation of the implantable active medical device (100) by a remote main writing device (200);
b) receiving (602), by the intermediate proximity device (110), writing instructions and data (205) sent via a network connection (300) by the remote main writing device (200);
c) placing a first non-implantable external unlocking tool (120) within a predetermined proximity perimeter of the implantable active medical device (100) for allowing (604) a radiofrequency communication (130) between the intermediate proximity device (110) and the implantable active medical device (100),
wherein the first unlocking tool (120) emits a signal to the implantable active medical device (100) that originates from an electromagnetic field or a static magnetic field or an inductive field;
d) writing (605) said writing data (205) into the memory (105) of the implantable active medical device (100) by the intermediate proximity device (110),
the method (600) being **characterized in that**:
step c) is preceded by a step of receiving, via the intermediate proximity device (110), the request to allow (603) the radiofrequency communication (130) between the intermediate proximity device (110) and the implantable active medical device (100) by means of a second non-implantable external unlocking tool (140),
wherein the second unlocking tool (140) comprises at least one of a password entered on the intermediate proximity device (110), a biometric input received by the intermediate proximity device (110), a magnetic card (141) read by the intermediate proximity device (110), a short-range wireless communication emitting a signal detectable by a proximity sensor of the intermediate proximity device (110), a magnetic field detected by the intermediate proximity device (110), or a transmission received via a peripheral port of the intermediate proximity device (110), in particular a USB port.

7. The method of claim 6, wherein the writing operation of the implantable active medical device (100) is prevented when the first unlocking tool (120) is not detected by the implantable active medical device (100), in particular when the distance (d2) between the first unlocking tool (120) and the implantable active medical device (100) is greater than 15 centimeters, more particularly greater than 10 centimeters.

8. The method of any one of claims 6 to 7, wherein the radiofrequency communication (130) between the implantable active medical device (100) and the intermediate proximity device (110) is carried out when said devices (100, 110) are separated by a distance of less than 10 meters.

9. The method of any one of claims 6 to 8, wherein, in step a), the request for a writing operation is signaled by a signaling means (115) of the intermediate proximity device (110) that emits a visual and/or audible and/or vibrating signal indicating that a writing operation is to be performed.

10. The method of any one of claims 6 to 9, wherein, in step a), the request for a writing operation is also sent, via a network connection, to a portable communication device (109), in particular a mobile phone, a tablet, a laptop, and/or a smartwatch, by means of a message, an SMS (Short Message Service), an email, or a similar type of alert.

11. The method of any one of claims 6 to 10, wherein the writing data (205) are stored by a storage means (113) of the intermediate proximity device (110) at least until a radiofrequency communication (130) between the intermediate proximity device (110) and the implantable active medical device (100) is allowed.
